(19) Europäisches Patentamt
European Patent Office
Office européen des brevets

(11) Publication number: **0 483 932 A1**

(12) **EUROPEAN PATENT APPLICATION**

(21) Application number: **91202836.2**

(51) Int. Cl.[5]: **C07D 295/14**

(22) Date of filing: **31.10.91**

(30) Priority: **31.10.90 HU 695390**

(43) Date of publication of application:
**06.05.92 Bulletin 92/19**

(84) Designated Contracting States:
**AT BE CH DE DK ES FR GB GR IT LI LU NL SE**

(71) Applicant: **RICHTER GEDEON VEGYESZETI GYAR R.T.**
**Gyömröi ut 19-21**
**H-1475 Budapest(HU)**

(72) Inventor: **Agai-Csongor, Eva**
**Rozália u. 35**
**HU-1031 Budapest(HU)**
Inventor: **Gizur, Tibor**
**Kassai u. 161/b**
**HU-1142 Budapest(HU)**
Inventor: **Harsányi, Kálmán**
**Fodor u. 12**
**HU-1126 Budapest(HU)**
Inventor: **Trischler, Ferenc**
**Uttöro u.16**
**HU-1171 Budapest(HU)**
Inventor: **Demeter-Szabo, Anik**
**Ady E. u. 97**
**HU-1204 Budapest(HU)**
Inventor: **Csehi, Attila**
**Lánchid u. 7**
**HU-2131 Göd(HU)**
Inventor: **Vajda, Eva**
**Semmelweis u. 9**
**HU-2510 Dorog(HU)**
Inventor: **Szab -Koml si, Györgyi**
**Arpád u. 7**
**HU-1183 Budapest(HU)**

(74) Representative: **Kupecz, Arpad et al**
**Octrooibureau Los en Stigter B.V. Postbox 20052**
**NL-1000 HB Amsterdam(NL)**

(54) **Process for the preparation of piperazine derivatives.**

(57) The invention relates to an improved process for the preparation of a piperazine derivative of the formula (I),

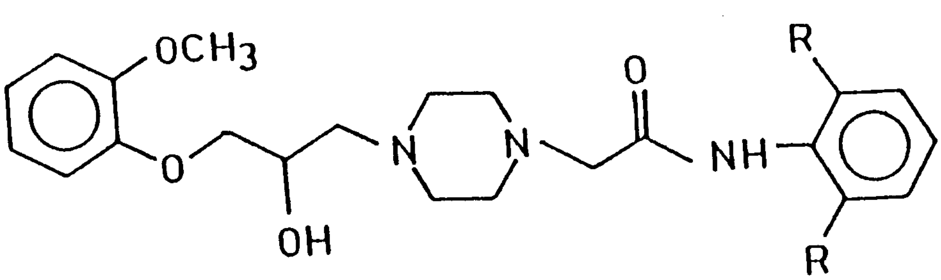

(I)

EP 0 483 932 A1

wherein R stands for a hydrogen atom or a methyl group, or a salt thereof.

According to the invention an amine compound of the formula (II)

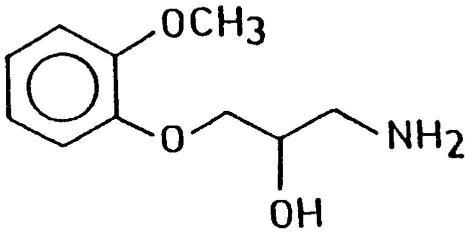

( II )

is reacted with a compound of the formula (III),

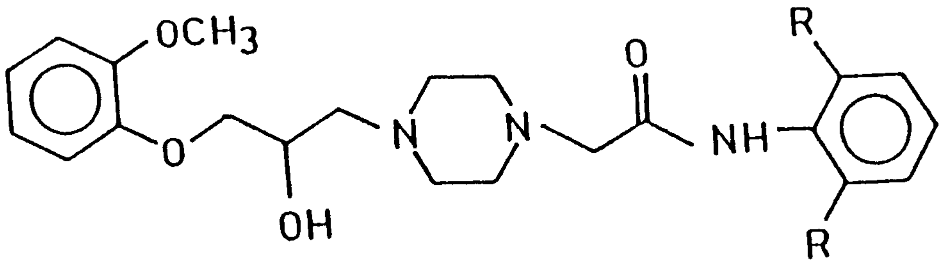

( I )

in which R is as defined above, or with an acid addition salt thereof.

The invention relates to a novel process for the preparation of piperazine derivatives and salts thereof. More particularly, the invention relates to the preparation of piperazine derivatives of the formula (I),

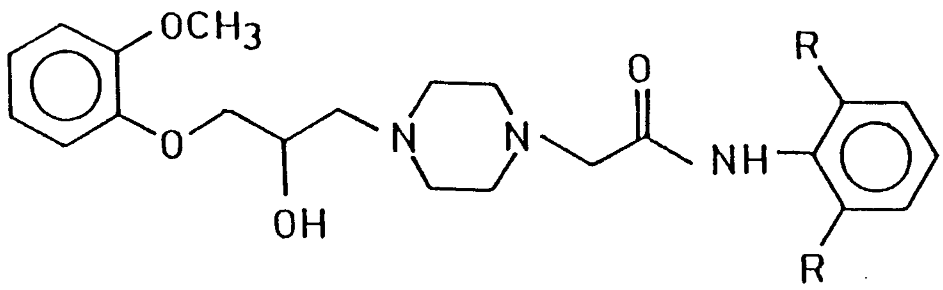

(I)

wherein R stands for a hydrogen atom or a methyl group, and salts thereof.

It is known that the compounds of the formula (I) influence the calcium-ion intake and can advantageously be used for the treatment of cardiovascular diseases such as myocardial infarction, congestive heart diseases, angina and arrhythmia.

The preparation of the above compounds is disclosed in the Hungarian patent specification No. 192,404. The authors describe two process variants. Both variants start from the same starting materials. The difference between the two process variants resides in the fact that the substituent groups on the two nitrogen atom of the piperazine ring are introduced in different sequences. The drawback of both variants is the linear synthesis route which is not advantageous in a more-step synthesis of this kind because the yields related to the starting materials are much too low at the end of the synthesis.

A further drawback of these processes is the necessity of using the piperazine in big excess in order to avoid the side-reaction on the other nitrogen atom of the ring.

The object of the invention is to provide a process for eliminating the above drawbacks and preparing the end products with high yields.

The invention is based on the recognition that if the compounds of the formulae (II) and (III) are prepared independently from each other by methods known per se and the piperazine ring is formed from these compounds as a closing step of the process, a pure and high quality end product is obtained with favourable yields.

Thus, the present invention relates to a process for the preparation of piperazine derivatives of the formula (I) and salts thereof - in the formula R stands for a hydrogen atom or a methyl group - by reacting an amine compound of the formula (II)

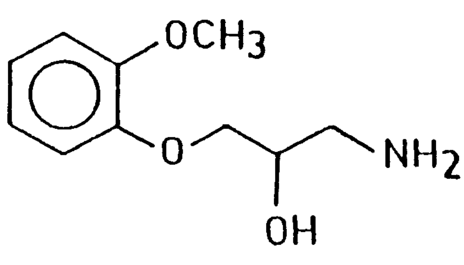

( II )

with a compound of the formula (III),

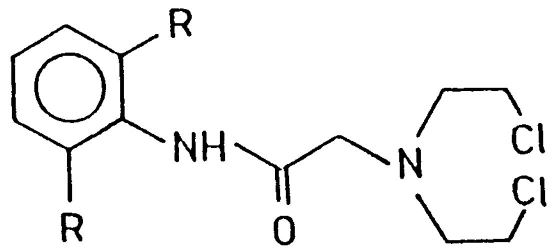

(III)

in which R is as defined above, or with an acid addition salt thereof.

According to a preferred embodiment of the process of the present invention the reaction is carried out in a water-miscible organic solvent, such as acetone. It is especially preferred to use as reaction medium a 30 to 60% by volume mixture of acetone and water.

The compound of the formula (II) is known and can be prepared e.g. from an appropriate oxirane derivative as described in J. Med. Chem. 9, 155 (1966).

The compound of the formula (III), in which R stands for hydrogen, is also known and can be prepared as described in Can. J. Chem. 45, 155 (1967) from N,N-bis(2-chloroethyl)amine-hydrochloride, or by reacting a corresponding bis(2-hydroxyethyl)-amino derivative with thionyl chloride. The compound of the formula (III), in which R stands for a methyl group, is novel. It can be prepared in an analoguous manner.

During the performance of the process of the present invention water and a water-miscible organic solvent are used as a solvent in the form of mixtures of different composition, preferably acetone containing 30 to 60% of water, at the boiling point. As an acid binding agent it is suitable to use different organic bases, preferably triethylamine, or an inorganic base, e.g. an aqueous solution of sodium hydroxyde.

According to a preferred embodiment of the invention the compound of the formula (III) or a hydrochloride salt thereof and the compound of the formula (II)used as starting materials are boiled in equimolar ratio, in the presence of triethylamine in an amount high enough for binding the acid formed, in a 50% aqueous acetone for 1 to 3 hours; then the acetone is removed by distillation and the product is extracted from the aqueous phase with an organic solvent. If desired, the product is subjected to chromatography, or it is purified by salt formation.

The advantages of the process of the invention can be summarized as follows:

- the process can be carried out in a simple way; and
- the starting material requirements can be lowered by preparing the two molecule parts independently from each other.

The process of the invention is elucidated by the following non-limiting examples.

Example 1

1-[3-(2-methoxyphenoxy)-2-hydroxypropyl]-4-(phenylaminocarbonylmethyl)piperazine dihydrochloride

0.82 g (0.003 mole) of α-[N,N-bis(2-chloroethyl)-amno]acetanilide hydrochloride, 20 $cm^3$ of 50% aqueous acetone, 0.6 g (0.003 mole) of 1-[3-(2-methoxyphenoxy)-2-hydroxypropyl]amine and 0.9 g (1.25 $cm^3$, 0.009 mole) of triethyl amine are weighed into a round bottom flask of a volume of 50 $cm^3$. The mixture is boiled for 2 hours, then cooled to room temperature and the acetone is distilled off in vacuo. The residual aqueous phase is extracted twice with 10 $cm^3$ of chloroform each. The organic phase is dried and evaporated. The residual oil is dissolved in 2 $cm^3$ of methanol and made acidic till pH 2 by adding methanolic hydrochloric acid. The precipitated crystalline title product is filtered and dried. Yield: 0.85 g (60.0%), melting point: 207-211 °C.

Example 2

a) α-[N,N-bis(2-hydroxyethyl)-amino]-2,6-dimethylacetanilide

7.9 g (0.04 mole) of [(2,6-dimethylphenyl)-aminocarbonylmethyl]chloride, 4.58 g (6.3 $cm^3$, 0.045 mole) of triethyl amine, 4.24 g (0.04 mole) of diethanol amine, 50 $cm^3$ of methyl isobutyl ketone and 0.1

g of sodium iodide are weighed into a round bottom flask of a volume of 100 $cm^3$. The mixture is boiled for 3 hours and filtered while hot. The filtrate is evaporated in vacuo to its half and cooled. The precipitated product is filtered and dried. Yield. 8.93 g (83.81%), melting point: 77-80 °C.

b) α-[N,N-bis(2-chloroethyl)-amino]-2,6-dimethylacetanilidehydrochloride

A three-necked round bottom flask of a volume of 250 $cm^3$ is equipped with a thermometer, a dropping funnel and a calcium chloride tube. 20 $cm^3$ of choloform and 10.76 g (6.6 $cm^3$, 0.09 mole) of thionyl chloride are weighed into it. A solution of 4.0 g (0.015 mole) of α-[N,N-bis(2-hydroxyethyl)-amino]-2,6-dimethylacetanilide in 20 $cm^3$ of chloroform are added under external cooling at a temperature of 5 °C. After terminating the addition the mixture is allowed to warm to room temperature and then it is stirred for 18 hours. Then the reaction mixture is cooled to 10 °C, 5 $cm^3$ of methanol are added and it is evaporated in vacuo on a bath of 30 °C. The residual oil is crystallized with ethyl acetate, then filtered and dried. Yield: 3.03 g (59.4%), melting point: 118 to 120 °C.

c) 1-[3-(2-methoxyphenoxy)-2-hydroxypropyl]-4-[(2,6-dimethyphenyl)-aminocarbonylmethyl)piperazine dihydrochloride

4.62 g (0.15 mole) of α-[N,N-bis(2-chloroethyl)-amino]-2,6-dimethylacetanilide, 40 $cm^3$ of 50% aqueous acetone, 3,0 g (0.015 mole) of 1-[3-(2-methoxyphenoxy)-2-hydroxy]-propylamine and 3.5 g (4.87 $cm^3$, 0.035 mole) of triethyl amine are weighed into a round bottom flask of a volume of 100 $cm^3$. The mixture is boiled for 2 hours and then the acetone is removed in vacuo. The residual aqueous suspension is extracted three times with 50 $cm^3$ of methylene chloride each. The extract is dried and evaporated. The thus obtained 4.88 g of oily product are subjected to chromatography on a column filled with 150 g of Kieselgel 60 (0.040-0.063 mm) as absorbent by eluating with a 9:1 mixture of chloroform and methanol. The eluates are evaporated to obtain 4.28 g of an oil which are dissolved in 15 $cm^3$ of methanolic hydrochloric acid and crystallized by adding diisopropyl ether. The precipitated product is filtered and dried. Yield: 4.35 g (58.0%), melting point: 229 to 230 °C.

## Claims

**1.** A process for the preparation of a piperazine derivative of the formula (I),

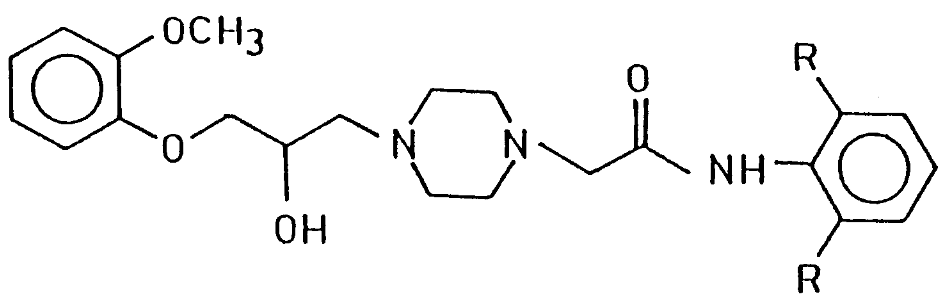

(I)

wherein R stands for a hydrogen atom or a methyl group, or a salt thereof, **characterized by** reacting an amine compound of the formula (II)

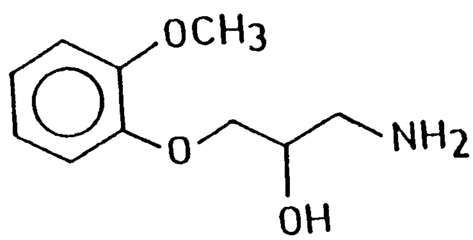

(II)

with a compound of the formula (III),

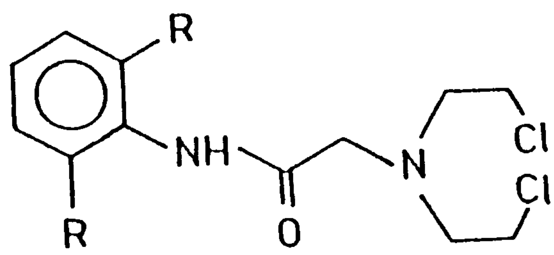

(III)

in which R is as defined above, or with an acid addition salt thereof.

**2.** A process as claimed in claim 1, **characterized by** carrying out the reaction in a water miscible organic solvent, preferably acetone.

**3.** A process as claimed in claims 1 or 2, **characterized by** using as reaction medium a 30-60% by volume mixture of acetone and water.

European Patent Office

# EUROPEAN SEARCH REPORT

Application Number

EP 91 20 2836

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | EP-A-0 126 449 (SYNTEX INC.) * claims * | 1-3 | C07D295/14 |
| Y | GB-A-662 283 (SOCIETE DES USINES CHIMIQUES RHONE-POULENC) * the whole document * | 1-3 | |
| | | | TECHNICAL FIELDS SEARCHED (Int. Cl.5 ) |
| | | | C07D |

The present search report has been drawn up for all claims

| Place of search | Date of completion of the search | Examiner |
|---|---|---|
| THE HAGUE | 30 JANUARY 1992 | PAUWELS G.R.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)